# EUROPEAN PATENT SPECIFICATION

(11) **EP 2 603 211 B1**
(45) Date of publication and mention of the grant of the patent: **28.09.2016**
(21) Application number: 11751960.3
(22) Date of filing: 10.08.2011
(51) Int. Cl.: A61K 31/40, A61K 36/28, A61P 3/06, A61K 45/06

(54) **COMPOSITION FOR TREATING HYPERLIPIDEMIA**
ZUBEREITUNG ZUR BEHANDLUNG DER HYPERLIPIDÄMIE
COMPOSITION POUR LE TRAITEMENT DE L'HYPERLIPIDEMIE

(30) Priority: 12.08.2010 NO 20101142
(43) Date of publication of application: 19.06.2013
(73) Proprietor: Naturmedical AS, 5131 Nyborg (NO)
(72) Inventor: STRØMMEN, Sigurd, N-5131 Nyborg (NO)
(74) Representative: Oslo Patentkontor AS
(86) International application number: PCT/NO2011/000222
(87) International publication number: WO 2012/021068

(56) References cited:
- CN-A- 1 899 342
- KÜÇÜKGERGIN CANAN ET AL: "Effect of artichoke leaf extract on hepatic and cardiac oxidative stress in rats fed on high cholesterol diet", BIOLOGICAL TRACE ELEMENT RESEARCH, HUMANA PRESS INC, NEW YORK, vol. 135, no. 1-3, 1 June 2010 (2010-06-01), pages 264-274, XP009152661, ISSN: 1559-0720, DOI: 10.1007/S12011-009-8484-9 [retrieved on 2009-08-04]
- MANGATHAYARU KALACHAVEEDU ET AL: "Modulatory effect of Inula racemosa Hook. f. (Asteraceae) on experimental atherosclerosis in guinea-pigs", JOURNAL OF PHARMACY AND PHARMACOLOGY, ROYAL PHARMACEUTICAL SOCIETY OF GREAT BRITAIN, GB, vol. 61, no. 8, 1 August 2009 (2009-08-01) , pages 1111-1118, XP009152660, ISSN: 0022-3573, DOI: 10.1211/JPP/61.08.0016 [retrieved on 2010-01-08]
- ASGARY S ET AL: "Antihypertensive and antihyperlipidemic effects of Achillea wilhelmsii", DRUGS UNDER EXPERIMENTAL AND CLINICAL RESEARCH, BIOSCIENCE EDIPRINT INC, CH, vol. 26, no. 3, 1 January 2000 (2000-01-01), pages 89-93, XP009152658, ISSN: 0378-6501
- ABID ZAKIA BEN ET AL: "Artemisia herba-alba Asso (Asteraceae) has equivalent effects to green and black tea decoctions on antioxidant processes and some metabolic parameters in rats", ANNALS OF NUTRITION AND METABOLISM: EUROPEAN JOURNAL OF NUTRITION, METABOLIC DISEASES AND DIETETICS, S. KARGER AG, SWITZERLAND, vol. 51, no. 3, 1 January 2007 (2007-01-01), pages 216-222, XP009152659, ISSN: 0250-6807
- Salika Aritajat et al.: "Effects of selected herbal extracts on blood chemistry profiles in rats", Southeast Asian J. Trop. Med. Public Health, vol. 39, no. suppl. 1 1 January 2008 (2008-01-01), 1 January 2008 (2008-01-01), pages 78-81, XP55009743, Retrieved from the Internet: URL:http://www.tm.mahidol.ac.th/seameo/200 8-39-suppl-1/13_2007.pdf [retrieved on 2011-10-17]
- ZHANG X F ET AL: "Effects of an ethanolic extract of Gynura procumbens on serum glucose, cholesterol and triglyceride levels in normal and streptozotocin-induced diabetic rats", SINGAPORE MEDICAL JOURNAL, ANDRE PUBLICATIONS, SINGAPORE, SG, vol. 41, no. 1, 1 January 2000 (2000-01-01), pages 9-13, XP009152662, ISSN: 0037-5675

## Description

### Ambit of the Invention

The present invention concerns a combinatory preparation for treating hyper-lipidemia/hyperliidemic disease and for hypercholesterolemia/hypercholesterolemic disease comprising whole, parts and/or a leeching or extract from the plant *Gynura divaricata* in combination with one of the compounds selected from the group ezetimib and rosuvastatin. The invention also concerns the use of the entire, parts of and/or a leech or extract of the plant *Gynura divaricata DC* of the family *Asteraceae* for producing a combinatory medication comprising additionally ezetimibe or rosuvastatin for producing a medication for treating hyperlipidemia and/or hyper-cholesterolemia. Such a medication may additionally include one or more exipients, diluents and/or carriers as well as colorants, taste additives, consistency-regulating substances and other non-active materials.

### Background for the Invention

Hyperlipidemic disease is caused by a number of genes disposing the afflicted person for high lipid values in the blood, e.g. high values of triglycerides and high values of cholesterol or the disease may have a foundation in the lifestyle of the afflicted person through the intake and digestion of food with high amounts of fat combined with an inactive way of living. When afflicted with hyperlipidemia the cholesterol values may be moderately elevated and are most often located about 6-8 mmol/l. The line of heritage may be that the hyperlipidemic disease appears randomly scattered in the family having as a consequence that not everyone in a family inherits the disposition for the disease.

One form of hyperlipidemia is hypercholesterolemia and particularly familial hyper-cholesterolemia. Familial hypercholesterolemia is a so-called dominant inheritable disease where there exists a high probability for inheriting the condition. The risk for hypercholesterolemia may be present if one of the parents has one healthy and one afflicted gene (so-called heterozygote) and the other parent is healthy, in which case there may exist a 50 % possibility for a child to inherit the disease, or if one of the parents has two afflicted genes (homozygote) and the other parent is healthy, in which case all the offspring become afflicted; or if both the parents possess an afflicted gene, in which case there is a 75 % chance for a child to inherit the disease; or if both the parents have afflicted genes, in which case all the children become ill (and additionally will become homozygote with a disposition for the disease being inheritable).

The disease is characterized in the formation of deposits of fat in the area around the eyes and along tendons. The disease is observed in 1 out of 400 persons, but represents 5 % of all the cases of infarction of the heart/stroke. These persons also have an elevated risk for blood vessel disease in the heart. In heterozygotes the total cholesterol values will frequently lie between 8 and 12 mmol/l. In homozygotes the values of the total cholesterol will frequently lie above 20 mmol/l. Normal cholesterol values lie conventionally below 6 mmol/l.

There may also appear a condition called familial combined hyperlipidemia. This condition is normally found in 1 of 50-100 persons. The disease gives an equally large risk for blood vessel disease of the heart as familial hypercholesterolemia. The total cholesterol is in this case normally 7-9 mmol/l and the triglyceride level lies above 2 mmol/l in the blood.

Additionally there may appear in humans a condition with elevated lipid values in the blood called metabolic syndrome. This is a condition wherein the normal total cholesterol, HDL cholesterol (HDL = High Density Lipoproteins) is reduced and the triglyceride values are elevated. At the same time these patients have an elevated blood pressure and a reduced tolerance for sugar, i.e. many develop diabetes. A phenotypic feature in such persons is the tendency of belly fat.

A number of diseases and medications may also contribute to the development of hyperlipidemia. This may be relevant for diabetes, affliction in the thyroid gland (hyperthyreosis), kidney and liver disease, a high intake of alcohol, the use of certain urinating medications, beta blockers may additionally contribute negatively and the same may be relevant for cortisone preparations and estrogens.

The diagnosing of hyperlipidemia/hypercholesterolemia may be conducted by determining the presence of such compounds in the blood and comprises the determination of total cholesterol, HDL cholesterol and triglycerides. The value of LDL (LDL = Low Density Lipoproteins) cholesterol may be calculated based on the value of these components in the blood. The measurement of blood glucose is conditioned to investigate if there is present a case of diabetes. In cases of cholesterol values above 8 mmol/l and isolated appearance of increased triglycerides, there is apart from the determination of blood sugar, also taken the levels of metabolite hormones, liver samples, kidney samples and the urine is examined for the appearance of proteins (kidney damage). In many cases there may also be conducted examination of the heart for signs of heart damage, e.g. by using ECG (electrocardiogram).

In cases where it is not sufficient or possible to treat hyperlipidemia with changes in lifestyle, the afflicted persons are treated medically. Normally used criteria for treating increased values of lipids/cholesterol in the blood is one or more or combinations of:
- Total cholesterol above 5.0 mmol/l
- A ratio between the values for total cholesterol and HDL cholesterol above 4
- LDL cholesterol above 3.0 mmol/l
- Triglyceride levels above 2.0 mmol/l
- HDL cholesterol below 1.0 mmol/l.

Examples of cases wherein treatment of increased values of lipids/cholesterol is recommended are:
- At cholesterol values between 6.5 -7.9 mmol/l and LDL cholesterol above 5.0 mmol/l or at a ratio between LDL and HDL of above 5
- A total cholesterol above 8 mmol/l
- The LDL cholesterol is continuously above 5 mmol/l or the ratio between LDL and HDL is continuously above 5.

Secondary diseases that may occur in hyperlipidemia are inter alia stroke and infarct/heart disease. This is caused by deposits of fat and cholesterol in the walls of blood vessels and capillaries. Such secondary diseases drain yearly society for large values since afflicted persons are on prolonged sick leave, become unable to work and have to use medications for having a normal life quality. The use of large quantities of medications, however, represents a strain on the afflicted persons both with respect to having to adapt to a rigid medicament regimen and also with respect to side effects when ingesting large amounts of or strong medications (or both). This is relevant both concerning treatment with one medication as well as a treatment with a combination of medicaments.

In relation to self-medication, medicaments taken orally are those that most easily make the recommended or prescribed medication to be performed. Consequently it is a further advantage to be able to present medications being more effective (than previously known medications against the same condition/disease) and that simultaneously may be taken orally. This is consequently relevant for oral administration forms such as pills, capsules, syrups, elixirs, powders, lozenges, etc. In self-medication it is a further advantage to be able to rely on the ingestion of as few units (pills etc.) of the medication as possible. To ingest a medication in a regimen comprising several units makes it possible to confuse which and how many pills that have been ingested, so there exists an increased risk for the medication per se to become erroneous. This is a drawback concerning e.g. elderly as well as partially demented persons or persons that habitually are easily distracted. Additionally a larger intake of pills is a strain per se for the patient to the extent that certain persons will sabotage the medication out of spite.

Consequently there is a large need for improving the existing medications by providing better working and more efficient compositions and combinations with weaker and/or less side effects and wherein the administration load is reduced.

### Prior Art

There are previously known several medications against elevated cholesterol (statins). Examples of such medications are "Ezetrol", "Liptor", "Crestor" and "Wellchol" and it may also be used "Tenormin" and "Albyl-E" or combinations of two or more thereof. These medications are present and are used as pills, and a continuous and/or high intake of such pills have several and more or less serious side effects while the strain of ingesting an arsenal of pills per se is deterrent and cumbersome for patients so in many cases there may be conducted a sloppy self-medication.

Concerning the plant *Gynura divaricata DC* preparations of this plant are known to have several effects.

Salika Aritajat et al.; "Effects of selected herbal extracts on blood chemistry profiles in rats", Southeast Asian J. Trop. Med. Public Health, vol 39, suppl. 1, p. 78-81, January 2008, discloses that Gynura divaricata extract reduces blood triglyceride levels. It is known from Chinese patent CN 101278957 a preparation from *Gynura divaricata DC* to be used in connection with the treatment of cancer. However, nothing concerning the treatment of hyperlipidemia or hypercholesterolemia is mentioned.

From Chinese patent CN 1899342 there are known oral formulations based on traditional Chinese medicine for treating diabetes, reduction of cholesterol in the blood and the prevention of hyperlipidemia. The preparation may be used in connection with other natural preparations (isoflavonoids from soy bean, flavonoids from the gingko leaf and all flavonoids from the kudzu root), but the combinational effect of these compositions has not been found to be specified.

From ABC Thesis Site (www.abclunwen.com/lunwen-free-301003/) People's Republic of China, Ministry of Information Industry, Net IPC, 2010.06.30 there are known chemical components from *Gynura divaricata DC,* inter alia hepatotoxic pyrrozilidine alkaloids reducing serum glucose, total cholesterol and triglycerides in test mice. Combination preparations with further lipid and cholesterol reducing substances are, however, not mentioned.

From an article of Zhang X. et al. (Singapore Medical Journal, vol. 41, no. 1, 2000) it is know that *Gynura procumbens* previously has been used for controlling diabetes mellitus and hyperlipidemia.

From an article by Gather, Health, "Garden Greens, the so-called spinaches", copyright 2010 Gather Inc. 2010.04.24, p. 1-3 it is known that the leaves from the plant *Gynura divaricata DC* have been used as tea with blood glucose reducing properties. *Gynura divaricata* is called Bai Bei San Qi in China and is used in connection with treating inter alia bronchitis, pulmonary tuberculosis and diabetes. Furthermore it is disclosed in this article that a spinach plant growing in the Maluku Islands in Indonesia (*Gynura divaricata* Dawn or Daun Dewa) is known from the folklore medicine to reduce cholesterol. However, this effect has not been substantiated or documented.

An article by K. Mangathayaru et al., Journal of Pharmacy and Pharmacology, vol. 61, no. 8, 2009, p. 1111-1118 discloses *Inula racemosa Hook* belonging to the family *Asteraceae* and the effects of different extracts on experimental atherosclerosis in pigs. Common for many of the plants belonging to this family, included *Inula racemosa,* is that they contain pyrrozilidine alkaloids. The results show that an alcoholic extract from the plant reduced total cholesterol, triglycerides and the LDL cholesterol while increasing the HDL cholesterol as compared to a positive control. There was not added any pyrrozilidine alkaloids beyond what was present in the plant.

From an article by Z. Ma et al., Zhongcaoyao, vol. 41, no. 4, 2010 concerning the hypoglycemic effects hypoglycemic effects of an aqueous extract from *Gynura divaricata (L.) DC* on rats with type 2 diabetes mellitus, it appears that the aqueous extracts significantly reduced the levels of inter alia blood glucose, total cholesterol and triglycerides and had a significantly therapeutic effect on type 2 diabetes mellitus.

The plant *Gynura divaricata* grows in the East (China, Thailand, etc.) and is a shrub with a green stem and grow-willing branches that in the winter ripen and have yellow flowers (Harborne et al., Phytochemical Dictionary: A Handbook of Bioactive Compounds from Plants, 2 nd ed. Padstow: TJ International, 1999:. P. 976). It is called Jakr-Na-Rai or Jin-Chee-Muo-Yea locally and is found in two types, namely a round-leafed type called Pae-Tum-Poung with soft light-green leaves covered with velvety hairs on both the dorsal and ventral surfaces and where the branches are red-green; and Jin-Chee-Muo-Yea having long leaves with a smooth surface and sparse hairs and that originates from China (but is also used in Thailand). The leaves and branch shoots are traditionally used for treating diabetes, hypertension, heart diseases, allergies, asthma, cancer, obesity, atherosclerosis, stomach diseases and kidney stone (Promrungraeng, M. et al., Cure all medicinal plant (March 19. 2007, to be found on http://www.navy.mi.th/navyboard/boarditem3.php).

### General Disclosure of the Invention

It has now surprisingly and conveniently been discovered that a composition of entire, sections of and/or a leeching or an extract of the plant *Gynura divaricata DC* of the family *Asteraceae* in combination with one or several other lipid and/or cholesterol-reducing substances i.e. ezetimibe and rosuvastatin provides a medication wherein the hyperlipidemic and hypercholesterolemic effect is synergistically increased or enhanced. Thus in one aspect the invention concerns the use of whole, sections of and/or a leeching or extract of the plant *Gynura divaricata DC* of the family *Asteraceae* in combination with ezetimibe and rosuvastatin for producing a medication with a synergistically increased or enhanced lipid and/or cholesterol-reducing effect for treating hyperlipidemia and/or hypercholesterolemia. Such a medication may additionally comprise one or more exipients, diluents and/or carriers as well as colorants, taste additives, consistency-regulating substances and other non-active materials or substances.

Carriers for the medications/combinations according to the present invention are such that conventionally are used for the relevant oral administration forms. E.g. there may be used liquid solutions comprising water and/or ethanol and/or edible fats and/or oils (water-in-oil emulsions or oil-in-water emulsions) all optionally containing different types of sweeteners (sugar types such as glucose, fructose sucrose, mannose, etc. and/or artificial sweeteners such as saccharose, xylitol, etc.). Furthermore it is possible to use conventional solid carriers and/or additives for dry oral medications (e.g. alum, calcium carbonate, etc. or mixtures of dry and moist additives for making soft tablets.

The medical combinations according to the present invention may also exist as capsules wherein such capsules may contain a mixture of each component, e.g. as dry powders or as lyophilized powders, or the capsules may be multi-compartment capsules wherein each medication is present separated by the aid of e.g. a septum. Such capsules may be made of e.g. gelatin (soft or hard) or some other type of edible polymer.

The basis for the present invention is the surprising, unexpected and favorable effect that a mixture of whole, parts of, an extract or a leeching of the plant *Gynura divaricate DC* together with ezetimibe and rosuvastatin provides a synergistically enhanced lipid- and cholesterol-reducing effect of such combinatory medications diminishing the need for taking excessive amounts of conventional corresponding medications. Said combination provides a combined and seemingly synergistic effect concerning the lowering of the lipid cholesterol values in the blood. This makes it possible to produce orally ingestible combinatory medications with an improved lipid and cholesterol-reducing effect comprising a mixture of said plant, plant parts, extract and/or leeching and/or dried parts and/or lyophilized powder together with one or more of the mentioned conventional cholesterol-reducing medications, than what was possible by using the prior state of the art. The presence of ezetimib and rosuvastatin as the conventional lipid and/or cholesterol-reducing medication may be reduced up to ten-fold as compared to the intake of the same medication without the presence of the extracted or leeched plant substance(s) according to the present invention.

The material from the plant *Gynura divaricata DC* may be present in the form indicated supra, i.e. in the form of whole or parts of the plant, dried plant or parts of the plant, an extract or a leeching from the plant or lyophilized plant, parts of the plant, leeching or extract, or as mixtures of these forms of the plant.

A further advantage with a combination preparation according to the invention is that the extract, leeching and/or powdered plant part provides an extraordinarily rapid cholesterol-reducing effect being lasting and that works in synergistic cooperation with the cholesterol-reducing effect of the ezetimibe and rosuvastatin as the conventional cholesterol-reducing medication. This has the effect that in addition to making it possible to reduce the intake or the amount of the conventional cholesterol-reducing medication(s), it will also speed up the cholesterol-reducing effect of the combinatory medication as compared to what was possible with the cholesterol-reducing medications(s) alone. Furthermore, this makes it possible to reduce the pill burden of the individual patient, reduce the side effects of the conventional medication(s) while providing a faster cholesterol-reducing effect of the combinatory medication according to the invention as compared to what was possible with the conventional preparation(s) alone.

Examples of materials from the plant *Gynura divaricata DC* to include in the combinatory preparation according to the invention are leaves, stems or shoots, possibly in dried form. Such plant parts, and particularly the leaves, may be dried and ground to a powder. This powder may be used directly as an additive to the conventional cholesterol-reducing substance(s) or may be used as a starting material for a leeching e.g. in the form of an aqueous leeching or an alcoholic leeching. It may also be possible to press or squeeze or pulp the plant to obtain a juice (e.g. by straining a mash of the plant through a cloth) and subsequently drying this extract (e.g. by air-drying at room temperature to avoid destroying the active components in the extract, or by lyophilizing the extract). If the above said powder or dried or fresh leaves or plant parts are leeched in water or alcohol (96% ethanol, 60% ethanol or 40% ethanol mixed with water) there will be formed a leeching that in its turn may be dried at room temperature or by lyophilizing, as mentioned supra. The leeching may be used as such as a component in the combinatory preparation according to the invention, e.g. as a liquid component incorporated in a carrier together with at least one of the ezetimib and rosuvastatin, or the dried powder may be used as a dry component in such a combinatory preparation.

One possible way to form a leeching/extract from the plant parts, especially the leaves, of the plant *Gynura divaricata DC* is to leech dried leaves or a powder of dried and ground leaves from the plant for an interval of 3 - 15 minutes, preferably 5 - 10 minutes, e.g. 5, 6, 7, 8 or 9 minutes in boiling water (100°C). This aqueous leeching/extract may be used as such as the synergistically working component in the combinatory preparation according to the invention (e.g. if the combinatory preparation is a mixture or elixir or some other form of liquid oral medication), or it may be dried as explained supra and used in a combinatory preparation according to the invention as a dry powder e.g. in a pill or capsule mixed with the at least one of the said other cholesterol-reducing conventional medication together with excipients and/or carriers.

The amount of plant preparation that is to be added to the relevant combinatory preparation according to the invention will relate to whether or not the relevant substance is a wet extract, a dried extract or a leeching or natural or dried plant parts (leaves). In relation to a powder of dried plant parts, an effective amount will be from 100 mg or more such as 200 mg or more, 500 mg or more, 800 mg or more, 1000 mg or more or 2000 mg or more. The upper limit to the plant substance additive is limited more on account of practical than medical considerations since an amount of 10 000 mg will give far too large an amount to be ingested easily, but the intake of such an amount has not been found to give any unwanted side effects medicinally. An amount in a combinatory preparation together with the other mentioned cholesterol-reducing substance(s) will give a reduction in the intake of the other conventional cholesterol-reducing substance of between 1 to 10 times (i.e. an intake of an amount of conventional medicine corresponding to 2 tablets may be lowered to an amount of the same substance corresponding to 1 tablet in a combination with the plant powder/extract mentioned supra and up to a conventional intake of a prior art cholesterol-reducing medication corresponding to 10 tablets may be reduced to an intake of an amount corresponding to 1 tablet by combining the conventional medicine with the plant powder/extract disclosed supra).

### Detailed Disclosure of the Invention

The combinatory preparation and the use according to the present invention will be more closely illuminated infra with reference to examples showing the combined effect of such a combinatory preparation. There will also be provided comparative examples illuminating the synergistic effect of the combinatory preparation according to the invention.

### Examples

### Example 1:

A male patient, 55 years of age, established hypercholesterolemia from the age of 39 with a cholesterol level in the blood of 13, was medicated daily since the diagnosis date for hypercholesterolemia with the medications "ezetrol" (10 mg, 1 tablet), "liptor" (10 mg, 1 tablet) and "wellchol" (625 mg, 6 tablets) giving a tablet load of 8 tablets daily. Despite this combinatory treatment the cholesterol levels of this person still remained between 6 and 7.5 (against a normal value of 5 or less).

As an alternative the medication "liptor" was replaced with the cholesterol-reducing substance "crestor" (10 mg, 1 tablet) without this having any effect on the cholesterol-reducing efficacy of the conventional preparations given to the patient.

Initially the test was conducted by the above specified conventional medication composition being further combined with the intake of 1 g ground and dried powder of the plant *Gynura divaricata DC.* This gave a rapid (1 month) and lasting reduction of the cholesterol values down to below 5 (normal level).

Furthermore, by combining the intake of 1 teaspoon (1 g) of dried leaves from the plant *Gynura divaricata DC* with the conventional cholesterol-reducing "crestor", it was possible to reduce the intake of tablets to 1 tablet daily (20 mg), and with this medication there were obtained lasting (1 year) cholesterol values in the blood of 4.3 to 4.7.

Synergy between the relevant plant material (from the plant *Gynura divaricata DC*) and the conventional cholesterol-reducing medications is presented as results from monthly cholesterol measurements taken during a period of 1 year with the above mentioned combination of conventional cholesterol-reducing medicines ("ezetrol", "liptor" and "wellchol"). The mean cholesterol value during the relevant period was 6.6. This value should for a normal person lie below 5 and is clearly too high despite a significant pill burden to the patient. The medication of the patient was changed (the combinations "Tenormin" (25 mg), "albyl-E" (160 mg), "ezetrol" (10 mg) and "crestor" (5 mg) as well as the combination "Tenormin" (25 mg), "albyl-E" (160 mg) and "ezetrol" (10 mg) without this having any effect on the mean cholesterol values in the blood.

The medication regimen of conventional cholesterol-reducing substances indicated supra was then taken together with the intake of 1 g of the plant extract indicated supra, rapidly reducing the cholesterol levels of the patient to below normal values, and it was even possible to reduce the pill burden of the patient to 1 tablet daily.

In the examples 2 - 5 infra there is shown the cholesterol-reducing effect of the intake of different forms of aqueous leeching and extracts or powders from the plant *Gynura divaricata DC* on hyperlipidemic persons. In example 2 the extract was produced by boiling ground leaves from *Gynura divaricata DC* for 5 minutes at 100°C. The residue from the leaves was filtered from the liquid through a cloth, and the liquid was taken as a tea as soon as the temperature had reached drinkable temperature (about 50°C or below). The examples are presented to show the very rapid cholesterol-reducing effect of such an extract as well as indicating the synergistic effect of the combination according to the invention since the plant extract alone had a cholesterol-reducing effect, but not as significant as when combined with the conventional cholesterol-reducing medications.

### Example 2:

A woman, age 29 years with established hypercholesterolemia (initial cholesterol value in the blood of 9,3) and without additional medication took an aqueous extract (2 dl water) from 1 g of crushed leaves from *Gynura* r *divaricata DC* once daily.

The cholesterol value was reduced to 7.9 after 40 days.

### Example 3:

A man, 57 years of age with established hypercholesterolemia (initial cholesterol values in the blood of 8.1) took 1 g of crushed leaves from *Gynura divaricata DC* as a dry powder once daily. The cholesterol value was reduced to 6.6 after 30 days.

### Example 4:

A man, 50 years of age with established hypercholesterolemia (initial cholesterol value in the blood of 8.0) took 1 g of ground leaves from *Gynura divaricata DC* as a powder once daily. The cholesterol value was reduced to 6.9 after 32 days.

### Example 5:

A woman, 55 years of age with established hypercholesterolemia (initial cholesterol value in the blood of 8.5) took a lyophilized preparation of 1 g from an aqueous leeching from 1 g of crushed leaves from *Gynura divaricata DC* produced as described in *Example* 2, wherein the leeching after cooling was lyophilized for forming a powder. This powder was taken once daily. The cholesterol value was reduced to 7.1 after 60 days.

### Conclusion:

The results from the examples supra show that the assimilation of a powder, an extract or a leeching made from dried leaves from the plant *Gynura divaricata DC* together with a conventional cholesterol-reducing medication including ezetimib and rosuvastatin, brings the values for cholesterol in the blood down after using such a combination over time. The reduction of the cholesterol values is lasting as long as the intake of such a medication is maintained. Furthermore, the results show that the cholesterol-reducing effect from the intake of different forms of the preparation from the plant *Gynura divaricata DC* initiates a very rapid cholesterol-reducing effect (between 1-2 months as compared to 2-3 months with conventional preparations). The cholesterol-reducing effect of the plant material alone, as shown in examples 2-5, does not reduce the cholesterol value to below the normal value of 5, but these results combined with the results from example 1, show that the effect of a combinatory preparation according to the invention both reduces the cholesterol values synergistically down to the normal value, that the cholesterol-reducing effect takes effect more rapidly than the known conventional medications and that the pill burden is significantly reduced in comparison to the state of the art.

## Claims

1. Combinatory preparation for treating hyperlipidemia and/or hypercholesterolemia, comprising whole, parts of and/or a leeching or extract from the plant *Gynura divaricata* in combination with one of the compounds selected from the group ezetimibe and rosuvastatin.

2. Combinatory preparation according to claim 1, wherein the whole, parts of and/or leeching and/or extract from the plant *Gynura divaricata* is combined with the compounds ezetimibe atorvastatin and colesevelam.

3. Combinatory preparation according to claim 1, wherein the whole, parts of and/or leeching and/or extract from the plant *Gynura divaricata* is combined with the compounds ezetimibe, rosuvastatin and colesevelam.

4. Combinatory preparation according to claim 1, wherein the whole, parts of and/or leeching and/or extract from the plant *Gynura divaricata* is combined with the compounds ezetimibe and colesevelam.

5. Combinatory preparation according to any of the claims 1 - 4, wherein the plant is of the type *Gynura divaricata DC* preferably of the type Pae-Tum-Poung and/or Jin-Chee-Muo-Yea.

6. Combinatory preparation according to any of the claims 1 - 5, wherein the parts of the plants are leaves.

7. Combinatory preparation according to claim 6, wherein the leaves are dried.

8. Preparation according to any of the preceding claims, wherein the leeching or extract from the plant is lyophilized.

9. Combinatory preparation according to any of the preceding claims, wherein the preparation comprises one or more substances selected from the group of excipients, diluents and/or carriers as well as colorants, taste additives, consistency-regulating substances and other non-active substances.

10. Combinatory preparation according to any of the preceding claims, wherein the preparation is present as an oral preparation, preferably a pill or a capsule.

11. Combinatory preparation according to any of the preceding claims, wherein the amount of plant parts and/or extract is 100 mg or more, e.g. 200 mg or more, 500 mg or more, 800 mg or more, 1000 mg or more or 2000 mg or more.

12. The use of whole, parts of and/or a leeching or extract from the plant *Gynura divaricata* together with one of the compounds selected from the group ezetimibe and rosuvastatin for making a combinatory medical preparation for treating hyperlipidemia and/or hypercholesterolemia.

## Patentansprüche

1. Kombinations-Zubereitung zur Behandlung von Hyperlipidämie und/oder Hypercholesterinämie, umfassend das Ganze, Teile und/oder einen Auszug oder Extrakt der Pflanze *Gynura divaricata* in Kombination mit einer der Verbindungen, ausgewählt aus der Gruppe Ezetimib und Rosuvastatin.

2. Kombinations-Zubereitung nach Anspruch 1, wobei das Ganze, Teile und/oder Auszug und/oder Extrakt der Pflanze *Gynura divaricata* mit den Verbindungen Ezetimib, Atorvastatin und Colesevelam kombiniert ist.

3. Kombinations-Zubereitung nach Anspruch 1, wobei das Ganze, Teile und/oder Auszug und/oder Extrakt der Pflanze *Gynura divaricata* mit den Verbindungen Ezetimib, Rosuvastatin und Colesevelam kombiniert ist.

4. Kombinations-Zubereitung nach Anspruch 1, wobei das Ganze, Teile und/oder Auszug und/oder Extrakt der Pflanze *Gynura divaricata* mit den Verbindungen Ezetimib und Colesevelam kombiniert ist.

5. Kombinations-Zubereitung nach einem der Ansprüche 1-4, wobei die Pflanze von der Art *Gynura divaricata DC,* vorzugsweise von der Art Pae-Tum-Poung und/oder Jin-Chee-Muo-Yea ist.

6. Kombinations-Zubereitung nach einem der Ansprüche 1-5, wobei die Teile der Pflanzen Blätter sind.

7. Kombinations-Zubereitung nach Anspruch 6, wobei die Blätter getrocknet sind.

8. Zubereitung nach einem der vorangehenden Ansprüche, wobei der Auszug oder der Extrakt der Pflanze lyophilisiert ist.

9. Kombinations-Zubereitung nach einem der vorangehenden Ansprüche, wobei die Zubereitung eine oder mehrere Substanzen, ausgewählt aus der Gruppe von Exzipienten, Verdünnungsmitteln und/oder Trägern sowie Färbemitteln, Geschmackszusätzen, Konsistenz-regulierenden Substanzen und anderen nicht-aktiven Substanzen, umfasst.

10. Kombinations-Zubereitung nach einem der vorangehenden Ansprüche, wobei die Zubereitung als eine orale Zubereitung, vorzugsweise eine Pille oder eine Kapsel vorliegt.

11. Kombinations-Zubereitung nach einem der vorangehenden Ansprüche, wobei die Menge der Pflanzenteile und/oder des Extrakts 100 mg oder mehr, z.B. 200 mg oder mehr, 500 mg oder mehr, 800 mg oder mehr, 1000 mg oder mehr oder 2000 mg oder mehr ist.

12. Verwendung des Ganzen, von Teilen und/oder einem Auszug oder Extrakt der Pflanze *Gynura divaricata* zusammen mit einer der Verbindungen, ausgewählt aus der Gruppe Ezetimib und Rosuvastatin, zur Herstellung einer medizinischen Kombinations-Zubereitung zur Behandlung von Hyperlipidämie und/oder Hypercholesterinämie.

## Revendications

1. Préparation sous forme de combinaison pour le traitement de l'hyperlipidémie et/ou l'hypercholestérolémie, comprenant la totalité, des parties de, et/ou un lixiviat ou un extrait de la plante *Gynura divaricata* en combinaison avec l'un des composés choisis dans le groupe constitué par l'ézétimibe et la rosuvastatine.

2. Préparation sous forme de combinaison selon la revendication 1, dans laquelle la totalité, des parties de, et/ou un lixiviat et/ou un extrait de la plante *Gynura divaricata* est combinée avec les composés ézétimibe, atorvastatine et colésévélam.

3. Préparation sous forme de combinaison selon la revendication 1, dans laquelle la totalité, des parties de, et/ou un lixiviat et/ou un extrait de la plante *Gynura divaricata* est combinée avec les composés ézétimibe, rosuvastatine et colésévélam.

4. Préparation sous forme de combinaison selon la revendication 1, dans laquelle la totalité, des parties de, et/ou un lixiviat et/ou un extrait de la plante *Gynura divaricata* est combinée avec les composés ézétimibe et colésévélam.

5. Préparation sous forme de combinaison selon l'une quelconque des revendications 1 à 4, dans laquelle la plante est du type *Gynura divaricata DC,* de préférence du type Pae-Tum-Poung et/ou Jin-Chee-Muo-Yea.

6. Préparation sous forme de combinaison selon l'une quelconque des revendications 1 à 5, dans laquelle les parties de la plante sont les feuilles.

7. Préparation sous forme de combinaison selon la revendication 6, dans laquelle les feuilles sont séchées.

8. Préparation sous forme de combinaison selon l'une quelconque des revendications précédentes, dans laquelle le lixiviat ou l'extrait de la plante est lyophilisé.

9. Préparation sous forme de combinaison selon l'une quelconque des revendications précédentes, dans laquelle la préparation comprend une ou plusieurs substances choisies dans le groupe constitué par les excipients, les diluants et/ou les vecteurs, aussi bien que les colorants, les arômes, les substances régulatrices de la consistance et autres substances non-actives.

10. Préparation sous forme de combinaison selon l'une quelconque des revendications précédentes, dans laquelle la préparation est présente en tant que préparation orale, de préférence en tant que pilule ou capsule.

11. Préparation sous forme de combinaison selon l'une quelconque des revendications précédentes, dans laquelle la quantité des parties et/ou extrait de plante est de 100 mg ou plus, par exemple 200 mg ou plus, 500 mg ou plus, 800 mg ou plus, 1000 mg ou plus ou 2000 mg ou plus.

12. Utilisation de la totalité, des parties de, et/ou d'un lixiviat ou d'un extrait de la plante *Gynura divaricata* conjointement avec l'un des composés choisis dans le groupe constitué par l'ézétimibe et la rosuvastatine pour la réalisation d'une préparation médicale sous forme de combinaison pour le traitement de l'hyperlipidémie et/ou l'hypercholestérolémie.
